# EUROPÄISCHE PATENTSCHRIFT

(11) **EP 1 144 587 B1**
(45) Veröffentlichungstag und Bekanntmachung des Hinweises auf die Patenterteilung: **23.07.2003**
(21) Anmeldenummer: 00906160.7
(22) Anmeldetag: 21.01.2000
(51) Int. Cl.: C12M 1/40, C12P 21/02

(54) **VORRICHTUNG ZUM ZELLFREIE SYNTHESE VON PROTEINE**
DEVICE FOR CARRYING OUT THE CELL-FREE SYNTHESIS OF PROTEINS
MODULE BIOREACTEUR SERVANT A REALISER DES REACTIONS BIOCHIMIQUES

(30) Priorität: 27.01.1999 EP 99101418
(43) Veröffentlichungstag der Anmeldung: 17.10.2001
(73) Patentinhaber: Roche Diagnostics GmbH, 68298 Mannheim (DE)
(72) Erfinder: SCHELS, Hans, D-80687 München (DE); MENZLER, Horst, D-82347 Bernried (DE); FISCHER, Ulrike, D-82377 Penzberg (DE)
(74) Vertreter: Pfeifer, Hans-Peter, Dr.
(86) Internationale Anmeldenummer: DE0000190
(87) Internationale Veröffentlichungsnummer: WO00044877

(56) Entgegenhaltungen:
- EP-A- 0 894 852
- WO-A-92/07062
- WO-A-94/18341
- US-A- 5 478 730
- KIM D M ET AL: "A semicontinuous prokaryotic coupled transcription/translation system using a dialysis membrane." BIOTECHNOLOGY PROGRESS., Bd. 12, Nr. 5, September 1996 (1996-09), Seiten 645-649, XP002106860 ISSN: 8756-7938

## Beschreibung

Die Erfindung betrifft einen Bioreaktionsmodul für die zellfreie Biosynthese von Proteinen oder anderen Polypeptiden. In der Biotechnologie werden biochemische Reaktionen vielfach in kleinen, leicht handhabbaren Reaktionsgefäßen durchgeführt, die hier als Bioreaktionsmodul bezeichnet werden. Sie dienen vor allem für die Biosynthese von Polypeptiden, wie beispielsweise Peptidhormonen, Antibiotika und - in neuerer Zeit besonders bedeutsam - Humanproteinen. Zu den dabei benutzten biochemischen Systemen gehören reine Translationssysteme, bei denen die Biosynthese auf dem genetischen Code einer Boten-RNA (mRNA) basiert und Transcriptions/Translations-Systeme, die auch den vorausgehenden Schritt der Bildung von Boten-RNA aus DNA umfassen.

Von besonderer Bedeutung ist die zellfreie Biosynthese, die im Vergleich zu in-vivo Systemen wesentliche Vorteile hat. Beispielsweise ermöglicht sie die Expression von toxischen und unstabilen Genprodukten. Die zellfreie Biosynthese wird oft für analytische Zwecke verwendet, insbesondere um rasch und bequem zu verifizieren, daß aus einem geklonten Gen tatsächlich die gewünschten Genprodukte synthetisiert werden. Daneben gibt es viele andere wichtige Anwendungsfälle, wie beispielsweise die Untersuchung von Eiweiß: Eiweiß Wechselwirkungen und Eiweiß:DNA Wechselwirkungen.

Ein Verfahren zur zellfreien Biosynthese von Polypeptiden ist beispielsweise in der US-Patentschrift 5,478,730 beschrieben. Das dort verwendete Translationssystem enthält eine Quelle von mRNA, die das Polypeptid codiert. Weiter sind in dem zellfreien Synthesesystem Ribosomen, tRNA, Aminosäuren, ATP und GTP enthalten. Die Translation der mRNA mit Hilfe der tRNA führt zur Produktion des Polypeptids, wobei zugleich Nebenprodukte und Abfallstoffe mit niedrigerem Molekulargewicht entstehen. Diese können durch eine semipermeable Membran, die den Raum, in dem das Synthesesystem enthalten ist, von einem Versorgungsraum trennt, in den Versorgungsraum übertreten. Der Versorgungsraum enthält eine als Versorgungsmedium wirkende Flüssigkeit, in der ATP, GTP und Aminosäuren enthalten sind. Diese Komponenten werden durch die semipermeable Membran dem Synthesesystem zugeführt, um den Verbrauch während der Bioreaktion zu ersetzen. Der Durchtritt durch die semipermeable Membran ist möglich, weil ihr Molekulargewicht unterhalb von deren Durchlaßgrenze liegt. Zugleich gelangen Produkte der biochemischen Reaktion und andere Substanzen, deren Molekulargewicht unterhalb der Durchlaßgrenze der Barriere liegt, aus dem Reaktionsraum in den Versorgungsraum. Die semipermeable Membran ist gemäß der US-Patentschrift 5,478,730 eine Ultrafiltrationsmembran in Form von Membran-Hohlfasern.

Die US-Patentschrift 5,478,730 enthält umfangreiche weitere Erläuterungen über geeignete Zusammensetzungen des Synthesesystems und der Versorgungsflüssigkeit. Insoweit bezieht sich die vorliegende Erfindung auf den vorbekannten Stand der Technik, wie er insbesondere dieser US-Patentschrift und den darin zitierten Literaturstellen zu entnehmen ist. Deren Inhalt wird durch Bezugnahme zum Inhalt der vorliegenden Anmeldung gemacht.

Die bei biochemischen Reaktionen insgesamt biochemisch wirksamen Komponenten kann man allgemein als "produzierendes System" bezeichnen. Ein produzierendes System kann grundsätzlich alles sein, was in der Lage ist, die Synthese gewünschter Verbindungen auf biochemischem Wege zu ermöglichen oder zu beschleunigen.

Da die für die Biosynthese von Polypeptiden erforderliche mRNA meist nur in sehr geringen Mengen zur Verfügung steht, sind die Bioreaktionsmodule vielfach sehr klein. Die Erfindung richtet sich insbesondere auf Mikro-Bioreaktionsmodule, bei denen die Kammer, die das produzierende System enthält, ("Systemkammer") ein Volumen von vorzugsweise höchstens 2 ml, jedenfalls weniger als 10 ml, hat. Da es sich dabei um zur einmaligen Verwendung vorgesehene Einwegprodukte handelt, kommt es besonders darauf an, daß solche Mikro-Bioreaktionsmodule zu günstigen Kosten herstellbar sind.

Ein besonderes Problem bei der Durchführung von biochemischen Reaktionen in Bioreaktionsmodulen besteht darin, eine möglichst hohe Ausbeute, also eine möglichst große Produktmenge bezogen auf die eingesetzte Menge an mRNA zu erzielen. Um dieses Ziel zu erreichen wird in der US Patentschrift 5,478,730 ein Verfahren beschrieben, das in der Fachwelt als CFCF (continuous flow cell free)-Synthese bezeichnet wird. Dabei wird das produzierende System in eine Ultrafiltrationszelle eingeschlossen und die Versorgungsflüssigkeit durch die semipermeable Membran hindurch in den Raum gepumpt, in dem sich das produzierende System befindet. Die Ausbeute ist dabei wesentlich von der pro Zeiteinheit dem produzierenden System zugeführten Menge an Versorgungsflüssigkeit (Zufuhrrate) abhängig. Beispielsweise wird in der US-Patentschrift 5,478,730 ein Versuch mit einem Synthesekammervolumen von 1 ml beschrieben, bei dem die Zufuhrrate zwischen 2 ml/h und 3 ml/h variiert wurde. Die Ergebnisse zeigen, daß mit der höheren Zufuhrrate eine hohe Produktionsleistung resultierte, während bei der niedrigeren Zufuhrrate die Produktmenge kaum zunahm.

Mit Hilfe des CFCF-Systems können hohe Produktausbeuten (über 100 µg für ein Systemkammervolumen von 1 ml) erreicht werden, jedoch sind damit erhebliche Nachteile verbunden.

Beispielsweise wird in der Publikation J. Davis et al.: "in vitro Transcription/Translation"; Promega Notes 56, 14 - 21, das CFCF-Verfahren wegen Problemen durch Verstopfung der Membran, störende Proteinaggregation und unerklärliche Unterbrechung der Translation kritisiert. Außerdem ist eine aufwendige Apparatur notwendig. Deshalb schlagen die Autoren dieser Publikation die Verwendung eines sogenannten "DispoDialyzer ^{R}" vor. Dabei handelt es sich um ein kleines röhrenförmiges Gefäß mit semipermeablen Wänden, in das 250 µl produzierendes System eingefüllt werden. Zur Durchführung der Biosynthese wird der DispoDialyzer in einem konischen 15 ml Laborröhrchen plaziert, das zwischen 3,5 und 7 ml Versorgungsflüssigkeit enthält. Das Laborröhrchen mit dem DispoDialyzer wird während der Reaktionszeit (20 Stunden) mittels eines Laborschüttelgerätes geschüttelt.

Eine ähnliche Experimentalordnung wird auch in der Publikation: "A Semicontinuous Prokaryotic Coupled Transcription/Translation System Using a Dialysis Membrane", von D.M. Kim et al. in Biotechnol. Prog., 1996, 645-649, beschrieben. Dabei wurde die Dialysemembran am unteren Ende eines zylindrischen Röhrchens mittels eines Ohrrings befestigt und das Röhrchen in einen Laborbecher eingetaucht, in dem die Versorgungsflüssigkeit mittels eines Magnetrührers gerührt wurde.

Durch diese Verfahrensweise sollen die Nachteile des CFCF-Verfahrens vermieden und es soll mit einer verhältnismäßig einfachen Apparatur eine hohe Produktausbeute ermöglicht werden. Diese Ziele werden jedoch nur in unzureichendem Umfang erreicht. Außerdem ist die Handhabung umständlich und die Qualität des Syntheseergebnisses sehr unterschiedlich. In vielen Fällen entstehen Undichtigkeiten oder sonstige mechanische Beschädigungen der Membran, die zu einem unbrauchbaren Resultat führen.

Aus der WO 94/18341 ist ein Verfahren zur zellfreien Synthese von Polypeptiden bekannt, durch das ebenfalls das CFCF-Verfahren verbessert werden soll. Dabei wird eine wiederverwendbare Bioreaktor-Flußzelle eingesetzt, bei der zwei Ultrafiltrationsmembranen in getrennten Gehäuseteilen enthalten sind. Das produzierende System wird mit einer Nährlösung gemischt und diese Mischung in eine Reaktionskammer des Bioreaktors eingeführt. Eine erhöhte Ausbeute soll dadurch erreicht werden, daß ein ganz bestimmter Strömungsweg eingehalten wird. Um dies zu gewährleisten, sind in den Gehäuseteilen Strömungskanäle als Vertiefungen eingeprägt. Die Ultrafiltrationsmembranen dienen dazu, die erzeugten Produkte abzutrennen, die zu zwei getrennten Eluat-Auslässen strömen.

Der Erfindung liegt die Aufgabe zugrunde, einen Bioreaktionsmodul zur Verfügung zu stellen, mit dem zellfreie Biosynthesen einfach, kostengünstig und mit einer möglichst hohen Ausbeute durchgeführt werden können.

Die Aufgabe wird gelöst durch einen Bioreaktionsmodul gemäß den unabhängigen Patentansprüchen. Bevorzugte Ausgestaltungen sind in den Unteransprüchen, bevorzugte Verfahrensweisen in den. Verfahrensansprüchen definiert.

Ein erfindungsgemäßer Bioreaktionsmodul besteht aus wenigen Teilen, die einfach hergestellt und zusammengebaut werden können. Trotz der daraus resultierenden geringen Herstellkosten ermöglicht er bei einfacher Handhabung die Durchführung von Bioreaktionen mit hoher Ausbeute.

Auf anderen Anwendungsgebieten sind bei dem erfindungsgemäßen Bioreaktionsmodul verwendete Konstruktionsmerkmale bekannt. Beispielsweise wird in der bereits erwähnten WO 94/18341 zur Abtrennung von radioaktiv oder fluoreszierend markierten Aminosäuren, die die Meßgenauigkeit beeinträchtigen würden, eine zwischen zwei Gehäuseteilen eingeklemmte Dialysemembran verwendet. In der WO 92/07062 wird im Zusammenhang mit einem Reaktor zur enzymatischen Behandlung von Wein oder Apfelwein die Möglichkeit beschrieben, als Alternative zu den bevorzugt verwendeten Hohlfaser-Membranen eine ebene semipermeable Membran einzusetzen.

Der erfindungsgemäße Bioreaktor eignet sich für unterschiedliche Bioreaktionsverfahren, darunter auch für Durchfluß(continuous flow)-Verfahren. Bevorzugt wird er jedoch bei Verfahren verwendet, bei denen kein Pumpvorgang stattfindet, d.h. die Versorgungsflüssigkeit in der Versorgungskammer während der biochemischen Reaktion nicht mit externem Druck beaufschlagt wird. Infolgedessen basiert der Substanzaustausch zwischen den Kammern ausschließlich auf Diffusion durch die Membran, also auf Dialyse. In dem Maß, in dem in der mittleren Kammer die Konzentration von bei der Biosynthese verbrauchten Substanzen abnimmt, strömen diese aufgrund des Konzentrationsgefälles aus der mindestens einen Versorgungskammer nach. Entsprechendes geschieht umgekehrt mit Abfallstoffen der Biosynthese.

Die semipermeable Membran ist bevorzugt eine Dialysemembran. Dialysemembranen sind (bei gleicher Ausschlußgrenze) im Verhältnis zu den bei Durchflußverfahren eingesetzten Ultrafiltrationsmembranen mechanisch weniger stabil. Dennoch treten bei dem erfindungsgemäßen Bioreaktionsmodul nur in sehr geringem Umfang mechanische Beschädigungen der Membran auf. Im Vergleich zu einer Ultrafiltrationsmembran mit gleicher Durchlaßgrenze wird eine wesentlich effektivere Biosynthese (bessere Ausbeute) erreicht. Bevorzugt liegt die Durchlaßgrenze (molecular cutoff) bei mindestens etwa 10 kD und höchstens etwa 20 kD.

Für manche Fälle ist eine Konstruktion vorteilhaft, bei der das Gehäuse zwei Versorgungskammern einschließt, die an eine Systemkammer angrenzen und von dieser durch eine erste und eine zweite semipermeable Membran getrennt sind. Das Gehäuse eines solchen 3-Kammer-Moduls weist zwei Versorgungskammerteile auf, wobei die erste semipermeable Membran zwischen dem Systemkammerteil und dem ersten Versorgungskammerteil und die zweite semipermeable Membran zwischen dem Systemkammerteil und dem zweiten Versorgungskammerteil eingespannt ist.

Gemäß einer weiteren bevorzugten Ausführungsform enthält mindestens eine, bevorzugt jede, Kammer des Moduls ein Magnetrührelement, das mittels eines externen Magnetfeldes in eine Drehbewegung versetzt werden kann. Der Bioreaktionsmodul wird als einmal verwendbares Wegwerfteil gebrauchsfertig, gegebenenfalls einschließlich Magnetrührelement, hergestellt.

Die Erfindung wird im folgenden anhand von in den Figuren dargestellten Ausführungsbeispielen näher erläutert; es zeigen:
- Fig. 1: Eine perspektivische Außenansicht eines erfindungsgemäßen Bioreaktionsmoduls,
- Fig. 2: eine Explosionsdarstellung des Moduls gemäß Fig. 1,
- Fig. 3: einen Querschnitt durch eine geringfügig abgewandelte Ausführungsform eines Bioreaktionsmoduls,
- Fig. 4: eine Detaildarstellung aus Fig. 3 zur Verdeutlichung einer bevorzugten Konstruktion zum Einspannen des Randbereiches einer Membran,
- Fig. 5: einen Querschnitt durch eine weitere bevorzugte Ausführungsform eines erfindungsgemäßen Bioreaktionsmoduls.

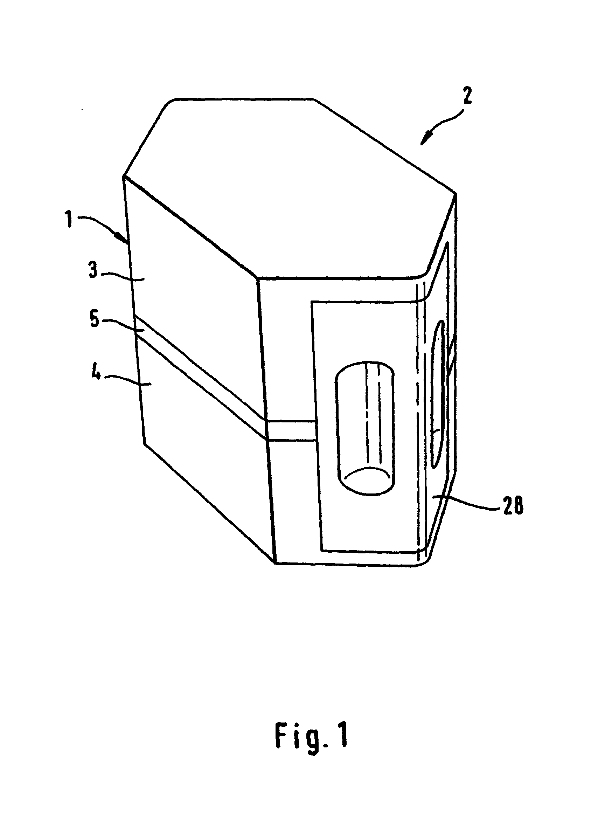

Das Gehäuse 1 des in den Figuren 1 bis 4 dargestellten Bioreaktionsmoduls 2 besteht im wesentlichen aus drei Kammerteilen, nämlich einem ersten Versorgungskammerteil 3, einem zweiten Versorgungskammerteil 4 und einem zwischen den beiden Versorgungskammerteilen angeordneten und mit diesen verbundenen Systemkammerteil 5. Eine erste semipermeable Membran 7 ist zwischen dem ersten Versorgungskammerteil 3 und dem Systemkammerteil 5 derartig eingespannt, daß ein peripherer, die Austauschfläche der Membran umgebender Randstreifen 14 zwischen den angrenzenden Kammerteilen 3,5 eingeklemmt und die Membran dadurch gespannt ist. In gleicher Weise ist zwischen dem zweiten Versorgungskammerteil und dem Systemkammerteil eine zweite semipermeable Membran 8 eingespannt.

Eine erste Versorgungskammer 10 wird durch die Wände einer in dem ersten Versorgungskammerteil 3 vorgesehenen nur zu dem Systemkammerteil 5 hin offenen Ausnehmung und die erste, die die Öffnung der Ausnehmung überspannende, semipermeable Membran 7 begrenzt. Entsprechend wird eine zweite Versorgungskammer 11 von den Wänden einer ebenfalls einseitig offenen Ausnehmung des zweiten Versorgungskammerteils 4 und der zweiten semipermeablen Membran umschlossen. Zwischen den beiden semipermeablen Membranen 7, 8 befindet sich eine Systemkammer 12, die von den beiden Membranen und den Wänden einer beidseitig offenen Ausnehmung des Systemkammerteils 5 gebildet wird. Die Wände der Ausnehmungen in den Kammerteilen 3,4 und 5 fluchten, d.h. ihre an die Membranen 7,8 angrenzenden Kanten umrahmen die Membranen entlang einer übereinstimmenden Begrenzungslinie.

Die Membranen 7,8 sind zwischen den Kammerteilen 3,5 bzw. 4,5 jeweils derartig dichtend eingespannt, daß ein molekularer Austausch zwischen der Systemkammer 12 und der benachbarten Versorgungskammer 10 bzw. 11 jeweils nur durch die Membran 7 bzw. 8 möglich ist. Dies wird durch an den Kammerteilen 3,5 bzw. 4,5 vorgesehene, die Austauschflächen 7a,8a der Membranen 7,8 umschließende ringförmige Dichtungsmittel 9 erreicht, zwischen denen der periphere Randbereich 14 der Membranen 7,8 dichtend eingespannt ist.

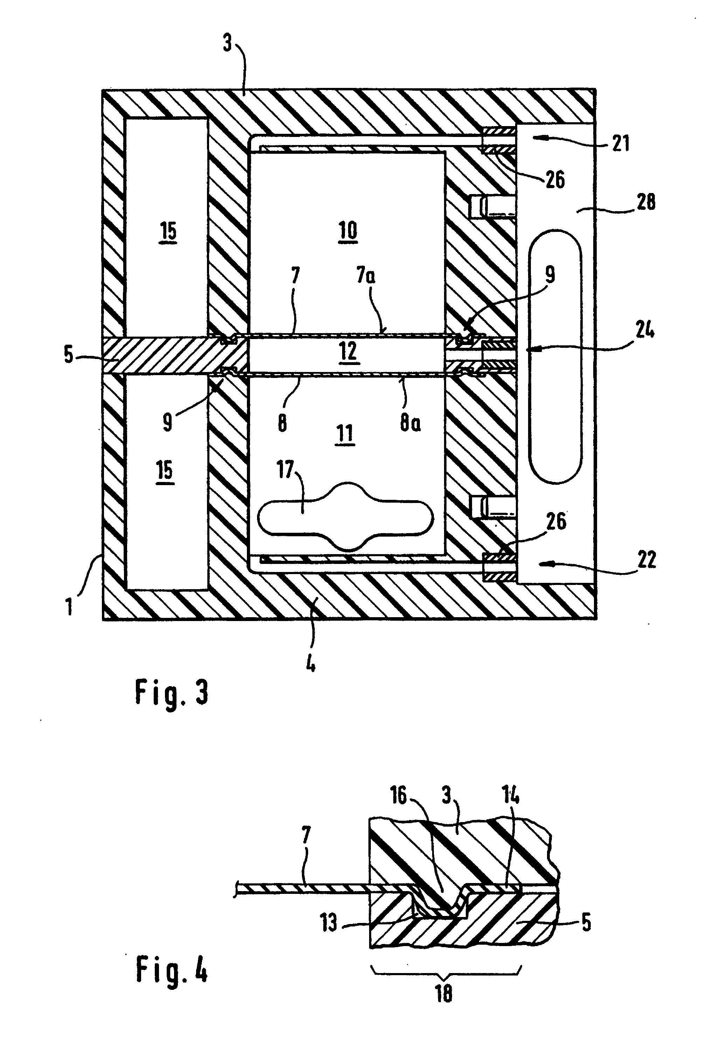

Eine bevorzugte Ausführungsform geeigneter Dichtungsmittel 9 ist in Figur 4 dargestellt. Dabei weist eines der die Membran einspannenden Kammerteile, im dargestellten Fall das Systemkammerteil 5, eine umlaufende Nut 13 und das andere Kammerteil, im dargestellten Fall das Versorgungskammerteil 3 eine mit der Nut fluchtende und in diese eindringende vorspringende Rippe 16 auf, durch die der zwischen den Kammerteilen 3,5 eingespannte periphere Randbereich 14 der Membran in die Nut 13 gedrückt wird. Der ringförmige Bereich der Kammerteile 3,5, in dem der periphere Randbereich 14 der Membran 7 eingespannt ist, wird als Spannbereich 18 bezeichnet. In einer praktisch bewährten Ausführungsform hat die Nut 13 eine Breite von etwa 1 mm und eine Tiefe von etwa 0,6 mm. Die Höhe der Rippe 16 beträgt dabei etwa 0,3 mm, ihre Fußbreite ist geringfügig kleiner als die Breite der Nut und ihre in die Nut eindringenden Kanten sind, wie in Figur 4 dargestellt, abgerundet.

Die Kammerteile 3,4,5 bestehen vorzugsweise aus Kunststoff, wie beispielsweise Polyether-Sulfon, PVC oder PMMA und werden beispielsweise als Gießteile hergestellt. Die Membran besteht aus Zelluloseacetat, unter Umständen aus Zellulosenitrat. In einem praktisch bewährten Ausführungsbeispiel betrug das Volumen der Systemkammer 1 ml und das Volumen der beiden benachbarten Versorgungskammern je 5 ml, so daß die Relation zwischen dem Volumen der Systemkammer und dem Gesamtvolumen der an die Systemkammer angrenzenden von dieser durch eine semipermeable Membran getrennten Versorgungskammern 1:10 betrug. Vorzugsweise liegt diese Relation bei mindestens 1:5 und höchstens 1:20, wobei Werte von mindestens 1:7 und höchstens 1:15 bevorzugt sind.

Die Verbindung der Kammerteile 3,4,5 kann mit in der Kunststofftechnik üblichen Mitteln, beispielsweise durch Schrauben, Kleben oder als Kunststoff-Steckverbindung erfolgen. Wesentlich ist, daß die Kammerteile 3 bis 5 eine ausreichende Steifigkeit haben, um einen gleichmäßigen Druck auf den Randbereich der Membranen zu gewährleisten. Zur Gewichtsersparnis und Verbesserung der Steifigkeit können die Kammerteile zusätzliche Hohlräume, die bei der Benutzung des Bioreaktionsmoduls nicht mit Flüssigkeit gefüllt werden, wie beispielsweise die in Figur 3 dargestellten Hohlräume 15, aufweisen.

Vorzugsweise enthält mindestens eine der Kammern ein Magnetrührelement 17, das der Übersichtlichkeit halber nur in einer der Versorgungskammern in Figur 3 dargestellt ist. Soweit mehrere Versorgungskammern vorhanden sind, ist es bevorzugt in beiden Versorgungskammern vorgesehen. Es kann auch vorteilhaft sein, in jeder der Kammern, also auch in der Systemkammer 12, ein Magnetrührelement 17 vorzusehen. In der Regel ist es bevorzugt, den Bioreaktionsmodul 2 möglichst weitgehend gebrauchsfertig, gegebenenfalls einschließlich der Magnetrührelemente, herzustellen und an den Benutzer zu liefern.

Die Verwendung eines Magnetrührelementes hat sich vor allem in Versorgungskammern mit einem Volumen von mehr als 2 ml, insbesondere mehr als 3 ml als vorteilhaft erwiesen. Bei noch kleineren Volumina der Versorgungskammer (und somit sehr kleinen Bioreaktionsmodulen) ist es vorteilhaft, den gesamten Bioreaktionsmodul während der biochemischen Reaktion zu schütteln. Dabei kann in mindestens einer der Kammern eine Rührkugel eingeschlossen sein, um die Durchmischung zu fördern.

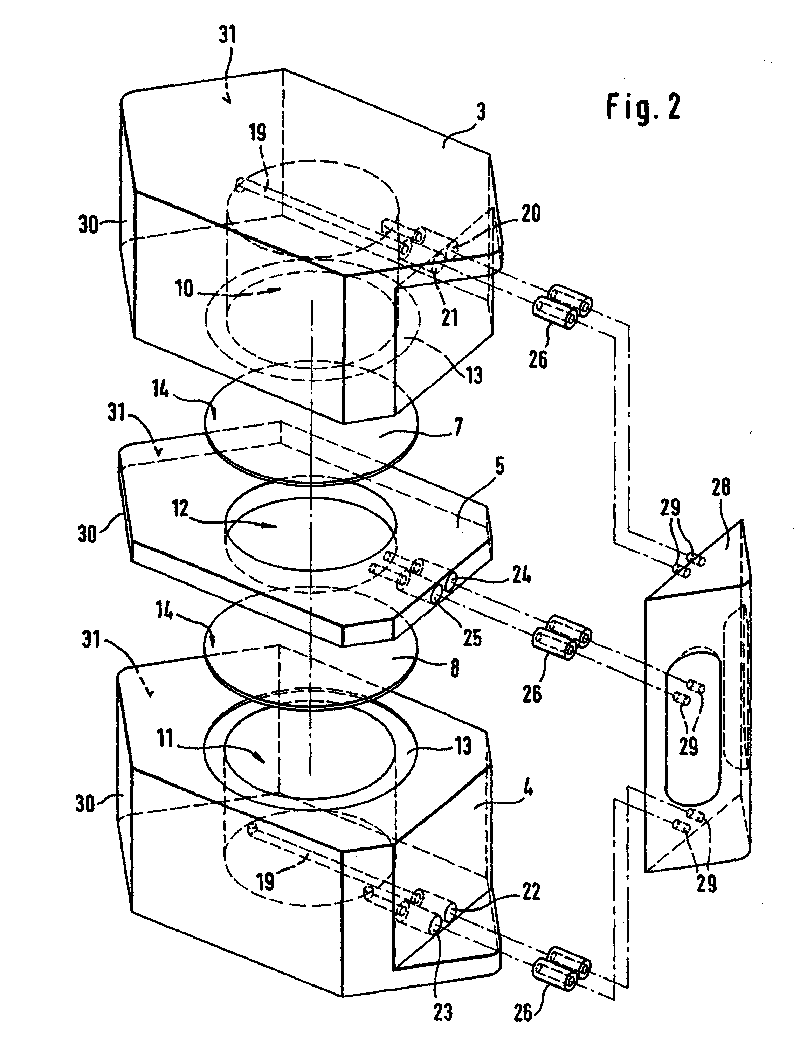

Die Kammern können mittels einer gewöhnlichen Laborpipette gefüllt werden. Zu diesem Zweck weisen sie jeweils Anschlußöffnungen auf, von denen Leitungen in die jeweiligen Kammern führen. Bei der in Figur 2 dargestellten Ausführungsform haben die Versorgungskammern 10,11 je zwei Anschlußöffnungen 20,21 bzw. 22,23 und auch die Systemkammer 12 hat zwei Anschlußöffnungen 24,25. Im Mündungsbereich der Anschlußöffnungen sind jeweils Dichtungen, beispielsweise zylinderförmige Silikongummidichtungen 26 vorgesehen, die ein dichtes Anschließen der Pipettenspitze ermöglichen. Die Anschlußöffnungen 20 bis 25 sind mittels eines Deckelteils 28 verschließbar, welches Verschlußzapfen 29 aufweist, die beim Aufsetzen des Deckelteils 28 in die Anschlußöffnungen eindringen und mittels der Dichtungen 26 einen dichten Verschluß bewirken.

Zur Durchführung einer biochemischen Reaktion werden die Versorgungskammern 10 und 11 mit einer Versorgungsflüssigkeit und die Systemkammer 12 mit einem produzierenden System gefüllt. Während der biochemischen Reaktion wird das mindestens eine Magnetrührelement 17 mittels eines externen Magnetfeldes in Rotation versetzt, so daß die Versorgungsflüssigkeit bzw. das produzierende System während der Reaktion ständig gerührt wird.

Für die Handhabung beim Befüllen des Bioreaktionsmoduls 2 ist es vorteilhaft, wenn das Gehäuse in einer Position aufgestellt werden kann, bei der die Anschlußöffnungen 20 bis 25 schräg nach oben gerichtet sind. Dies läßt sich dadurch erreichen, daß das Gehäuse eine quer zu der Membranoberfläche verlaufende Fußebene aufweist, die so orientiert ist, daß dann, wenn der Modul sich in einer Füllposition befindet, bei der er mit in der Fußebene liegenden Abstützpunkten auf einer horizontalen Fläche steht, die Membran im wesentlichen vertikal verläuft, wobei sich die Anschlußöffnungen 20 bis 25 im oberen Teil des Moduls, also zumindest oberhalb von dessen Mittelpunkt befinden. Diese Position wird als Füllposition bezeichnet. Bei dem in Figur 2 dargestellten Modul bilden die schräg verlaufenden Flächen 30 und 31 des insgesamt rombisch gestalteten Gehäuses jeweils eine Fußebene, die diese Bedingungen erfüllt. Die Fußebene muß jedoch nicht von einer geschlossenen ebenen Gehäusefläche gebildet werden. Da es nur auf die Orientierung des Gehäuses 1 ankommt, genügen drei Abstützpunkte, die eine die genannte Bedingung erfüllende Fußebene bilden.

Zum Füllen ist es vorteilhaft, wenn mindestens die Versorgungskammern jeweils eine Leitung 19 aufweisen, die dann, wenn sich der Modul in der Füllposition befindet, von der jeweiligen Anschlußöffnung derartig in den unteren Bereich der jeweiligen Kammer 10 bzw. 11 führen, daß sich die Mündung im unteren Bereich der Kammer, zumindest unterhalb von deren Mittelpunkt befindet.

Obwohl die Erfindung anhand eines 3-Kammer-Moduls beschrieben wurde, sind - wie dargelegt - auch Ausführungsformen mit einer abweichenden Anzahl von Kammern, insbesondere ein 2-Kammer-Modul, möglich. Die Konstruktion der Kammerteile und der Membraneinspannung kann dabei ähnlich wie bei den obigen Figuren gestaltet sein, wobei aber selbstverständlich das Systemkammerteil einseitig (auf der von der Versorgungskammer abgewandten Seite) geschlossen ist.

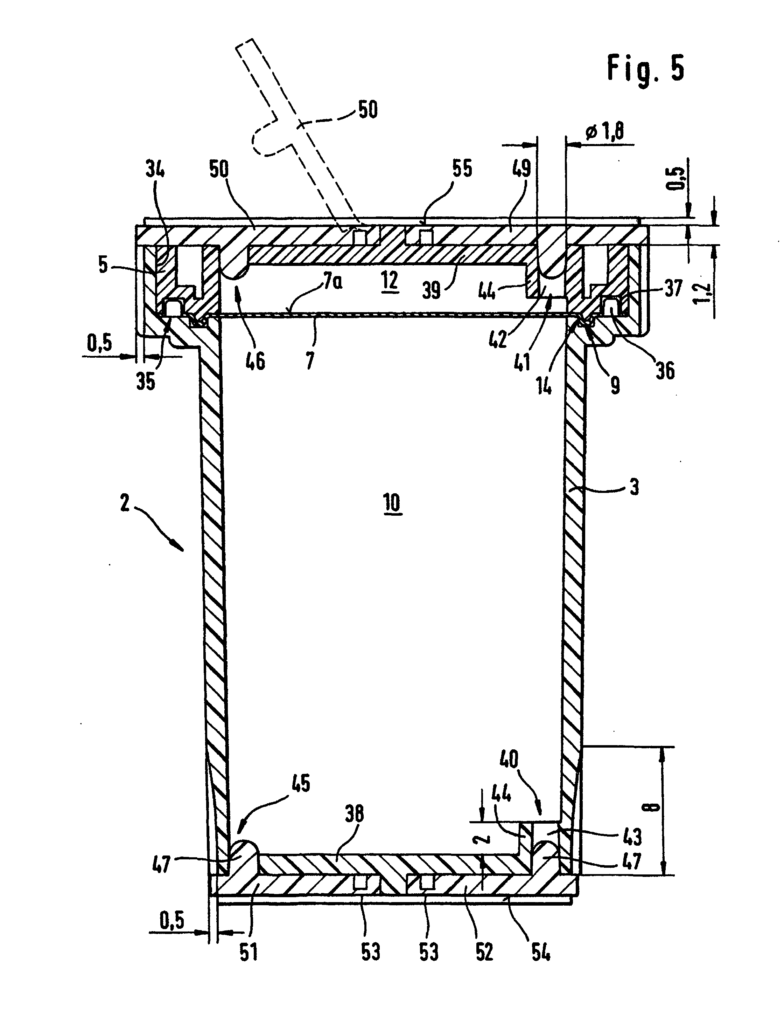

In Figur 5 ist ein solcher 2-Kammer-Modul dargestellt. Dabei sind die in ihrer Funktion der bisher beschriebenen Ausführungsform entsprechenden Bauteile mit den gleichen Bezugsziffern bezeichnet. Der Bioreaktionsmodul 2 enthält eine Systemkammer 12 und eine Versorgungskammer 10. Beide Kammern sind durch eine semipermeable Dialysemembran 7 voneinander getrennt. Die Systemkammer 12 ist von einem Systemkammerteil 5 und der semipermeablen Membran 7 begrenzt. Entsprechend ist die Versorgungskammer 10 von einem Vorsorgungskammerteil 3 und der semipermeablen Membran 7 begrenzt. Die Membran 7 ist an ihrem peripheren Randbereich 14 mittels ringförmig umlaufenden Dichtungsmitteln 9 dichtend eingespannt, wobei bevorzugt die anhand von Figur 4 beschriebene Konstruktion verwendet wird.

Zur Zentrierung und Fixierung der Membran 7 sind bei der dargestellten Ausführungsform mehrere (beispielsweise sechs) über den Umfang verteilte an einem der Kammerteile (hier dem Versorgungskammerteil 3) angegossene Stifte 36 vorgesehen. Die Membran 7 weist mit den Stiften 36 fluchtende Löcher 35 auf. Durch eine leicht konische Gestaltung der Stifte 36 und eine entsprechende Dimensionierung und Positionierung der Löcher 35 wird erreicht, daß die Membran 7 beim Einsetzen in das mit den Stiften versehene Kammerteil 3 leicht vorgespannt wird.

Die Verbindung der Kammerteile 3 und 5 erfolgt bei der dargestellten bevorzugten Ausführungsform dadurch, daß das Systemkammerteil 5 in eine entsprechende vertiefte Aufnahme 34 des Versorgungskammerteils 3 eingepresst wird. Um eine stabile Befestigung zu gewährleisten, ist der Außendurchmesser des Systemkammerteils 5 mit einem geringfügigen Übermaß im Vergleich zu dem Innendurchmesser der Aufnahme 34 gefertigt.

An jedem der Kammerteile 3,5 ist jeweils in einer der Membran 7 gegenüberliegenden Wand 38 bzw. 39 eine Füllöffnung 40 bzw. 41 zum Füllen der von dem jeweiligen Kammerteil umschlossenen Kammer (also der Versorgungskammer 10 bzw. der Systemkammer 12) vorgesehen. Die Füllöffnungen 40,41 haben vorzugsweise jeweils einen runden Querschnitt und gehen in einen Füllkanal 42,43 über, der von einer rohrförmigen Wand 44 begrenzt wird, die sich in die jeweilige Kammer 10 bzw. 12 hinein erstreckt.

Der Füllkanal dient vor allem dem Zweck, das Schäumen der in die Kammer einzufüllenden biochemischen Flüssigkeit zu reduzieren. Der dargestellte Bioreaktionsmodul ist für Fälle vorgesehen, bei denen nur die in die Systemkammer 12 einzufüllende Flüssigkeit zur Schaumbildung neigt. Deswegen führt dort der Füllkanal 42 bis nahe an die Membran 7. Der in der Versorgungskammer 10 vorgesehene im Verhältnis zu der Länge der Versorgungskammer sehr kurze Füllkanal 43 eignet sich zum Einfüllen einer nichtschäumenden Flüssigkeit. Für Fälle, bei denen auch die Versorgungsflüssigkeit zur Schaumbildung neigt, wäre auch der Füllkanal 43 bis nahe an die Membran 7 heranzuführen.

Ebenfalls in den der Membran 7 gegenüberliegenden Wänden 38,40 ist jeweils eine Entlüftungsöffnung 45 bzw. 46 vorgesehen. Sie hat vorzugsweise eine Gestalt (beispielsweise in Form eines sehr schmalen Langloches), die das Ansetzen einer Pipette unmöglich macht, so daß versehentliches Befüllen durch die Belüftungsöffnung nicht möglich ist.

Die Füllöffnungen 40,41 und die Entlüftungsöffnungen 45,46 sind jeweils mittels eines Verschlußstopfens 47 verschließbar, der jeweils von einem Klappteil 49 bis 52 senkrecht zu dessen Flächenausdehnung hervorspringt. Die Klappteile 49 bis 52 sind - kostengünstig mittels Filmscharnieren 53 - jeweils an der der semipermeablen Membran 7 gegenüberliegenden Wand 38 bzw. 39 derartig schwenkbar angelenkt, daß der Stopfen 47 in die jeweilige Öffnung 40,41,45,46 dichtend eindringt, wenn das Klappteil 49 bis 52 in die in Figur 5 dargestellte Position gebracht wird. Zum Öffnen der jeweiligen Öffnung wird das Klappteil hochgeschwenkt, wie dies beispielsweise für das Klappteil 50 gestrichelt dargestellt ist.

Auch bei der in Figur 5 dargestellten Ausführungsform befinden sich in den Kammern 10,12 vorzugsweise (der Übersichtlichkeit halber nicht dargestellte) Magnetrührelemente, die bei der Herstellung des Moduls 2 in die Kammern eingebracht wurden, so daß der gebrauchsfertig an den Benutzer gelieferte Modul die Magnetrührelemente 17 vorgepackt enthält.

Die erfindungsgemäßen Bioreaktionsmodule ermöglichen eine sehr effektive biochemische Reaktion mit einer vergleichsweise geringen Austauschfläche 7a der Membran 7 in Relation zu dem Volumen der jeweiligen Systemkammer 12.

Vorzugsweise liegt die Relation zwischen der Gesamtaustauschfläche von einer oder mehreren die Systemkammer 12 gegenüber einer oder mehreren Versorgungskammern begrenzenden Membranen und dem Gesamtvolumen der Systemkammer 12 bei höchstens 4,0 Einheit⁻¹, wobei eine Untergrenze von 0,2 Einheit⁻¹ nicht unterschritten werden sollte.

Der in Figur 5 dargestellte Bioreaktionsmodul ermöglicht eine besonders einfache Handhabung ohne Einschränkungen der Effektivität. Er ist sehr klein, wie die in Figur 5 eingetragenen Maßangaben (in mm) verdeutlichen. Zweckmäßigerweise bilden die Wände 38,39, die die Füllöffnungen 40,41 und vorzugsweise auch die Entlüftungsöffnungen 45,46 enthalten, jeweils eine Standfläche 54 bzw. 55, die so ausgebildet und orientiert ist, daß die jeweils andere Kammer 12 bzw. 10 befüllt werden kann, wenn der Modul 2 auf einer eine der Kammern 10 bzw. 12 begrenzenden Standfläche 54 bzw. 55 steht.

Bei einem Modul mit nur zwei Kammern ist die dargestellte Gestaltung mit zwei im wesentlichen parallel verlaufenden einander gegenüberliegenden Flächen 38,39, darin enthaltenen Öffnungen 40,41,45,46 und den entsprechen Klappteilen 49 bis 52 besonders vorteilhaft. Die genannten Konstruktionselemente sind aber auch bei anderen Gestaltungen des Bioreaktionsmoduls 2 vorteilhaft verwendbar.

## Patentansprüche

1. Einweg-Bioreaktionsmodul zur einmaligen Verwendung für die zellfreie Polypeptid-Biosynthese,
mit einem Gehäuse (1), das eine Systemkammer (12) und eine Versorgungskammer (10) umschließt, wobei die Systemkammer (12) während der biochemischen Reaktion ein produzierendes System enthält, die Versorgungskammer (10) während der biochemischen Reaktion eine Versorgungsflüssigkeit enthält und die Systemkammer (12) und die Versorgungskammer (10) durch eine semi-permeable Membran (7) getrennt sind,
bei welchem das Gehäuse (1) mindestens zwei Kammerteile (3,5) mit je einem ringförmigen Spannbereich (18) aufweist und zwischen den ringförmigen Spannbereichen (18) der Kammerteile (3,5) eine semipermeable Membran (7) mittels Dichtungsmitteln (9), die Membran (7) an ihrem peripheren Randbereich (14) einfassen , derartig abdichtend gespannt ist, daß die Systemkammer (12) von einem Systemkammerteil (5) und der semipermeablen Membran (7) und die Versorgungskammer von einem Versorgungskammerteil (3) und der semipermeablen Membran (7) begrenzt ist.

2. Modul nach Anspruch 1, bei welchem eines der Kammerteile (5 bzw. 3) in dem zum Einspannen der semipermeablen Membran (7) dienenden Spannbereich (18) eine umlaufende Nut (13) und das andere Kammerteil (3 bzw. 5) eine in die Nut (13) eindringende vorspringende Rippe (16) aufweist, durch die die Membran (7) an ihrem zwischen den Kammerteilen (3,5) eingespannten peripheren Randbereich (14) in die Nut gedrückt wird.

3. Einweg-Bioreaktionsmodul zur einmaligen Verwendung für die zellfreie Polypeptid-Biosynthese,
mit einem Gehäuse (1), das eine Systemkammer (12) und eine Versorgungskammer (10) umschließt, wobei die Systemkammer (12) während der biochemischen Reaktion ein produzierendes System enthält, die Versorgungskammer (10) während der biochemischen Reaktion eine Versorgungsflüssigkeit enthält und die Systemkammer (12) und die Versorgungskammer (10) durch eine semi-permeable Membran (7) getrennt sind,
bei welchem
das Gehäuse (1) mindestens zwei Kammerteile (3,5) aufweist, zwischen denen eine semipermeable Membran (7) mittels die Membran (7) an ihrem peripheren Randbereich (14) einfassenden Dichtungsmitteln (9) derartig abdichtend gespannt ist, daß die Systemkammer (12) von einem Systemkammerteil (5) und der semipermeablen Membran (7) und die Versorgungskammer von einem Versorgungskammerteil (3) und der semipermeablen Membran (7) begrenzt ist und
die mindestens eine Versorgungskammer (10) und/oder die Systemkammer (12) ein Magnetrührelement (17) enthält.

4. Modul nach einem der vorhergehenden Ansprüche, bei welchem mindestens eines der Kammerteile (3,5) in einer der semipermeablen Membran (7) gegenüberliegenden Wand (38,39) eine Füllöffnung (40,41) zum Füllen der von dem Kammerteil (3,5) umschlossenen Kammer (10,12) aufweist.

5. Modul nach Anspruch 4, bei welchem sowohl das Systemkammerteil (5) als auch das Versorgungskammerteil (3) in einer der Membran gegenüberliegenden Wand (38,39) eine Füllöffnung (40,41) zum Füllen der von dem Kammerteil (3,5) umschlossenen Kammer (10,12) aufweist.

6. Modul nach Anspruch 5, bei welchem die die Füllöffnungen (40,41) enthaltenen Wände (38,39) des Systemkammerteils (5) und des Versorgungskammerteils (3) jeweils als Standflächen (54,55) für den Modul (2) ausgebildet und derartig orientiert sind, daß bei auf einer solchen Standfläche (z.B. 54) stehendem Modul die jeweils andere Kammer (z.B. 12) befüllt werden kann.

7. Modul nach einem der Ansprüche 4 bis 6, bei welchem die Füllöffnung (40,41) mittels eines Verschlußstopfens (47) verschließbar ist, der von einem Klappteil (49-52) hervorspringt, das an der der semipermeablen Membran (7) gegenüberliegenden Wand (38,39) schwenkbar angelenkt ist.

8. Modul nach einem der Ansprüche 4 bis 7, bei welchem die der semipermeablen Membran (7) gegenüberliegende Wand (38,39) außer der Füllöffnung (40,41) eine zusätzliche Entlüftungsöffnung (45,46) aufweist.

9. Modul nach einem der vorhergehenden Ansprüche, bei welchem die semipermeable Membran (7) eine Dialysemembran ist.

10. Modul nach einem der vorhergehenden Ansprüche, bei welchem die Ausschlußgrenze der Membran (7) mindestens 10 kDalton und höchstens 20 kDalton beträgt.

11. Modul nach einem der vorhergehenden Ansprüche, bei welchem das Gehäuse eine Systemkammer (12) und zwei an die Systemkammer angrenzende Versorgungskammern (10,11) einschließt, die von der Systemkammer (12) durch eine erste und eine zweite semipermeable Membran (7,8) getrennt sind und das Gehäuse (1) zwei Versorgungskammerteile (3,4) aufweist, wobei die erste semipermeable Membran (7) zwischen dem Systemkammerteil (5) und dem ersten Versorgungskammerteil (3) und die zweite semipermeable Membran (8) zwischen dem Systemkammerteil (5) und dem zweiten Versorgungskammerteil (4) eingespannt ist.

12. Modul nach einem der vorhergehenden Ansprüche, bei welchem die Relation zwischen dem Volumen der Systemkammer (12) und dem Gesamtvolumen von einer oder mehreren an die Systemkammer (12) angrenzenden, von dieser durch eine semipermeable Membran getrennten Versorgungskammern (10,11) mindestens 1:5 und höchstens 1:20 beträgt.

13. Modul nach einer der vorhergehenden Ansprüche, bei welchem die Relation zwischen der Gesamtaustauschfläche von einer oder mehreren die Systemkammer gegenüber einer oder mehreren Versorgungskammern begrenzenden Membranen und dem Gesamtvolumen der Systemkammer mindestens 0,2 Einheit⁻¹ und höchstens 4,0 Einheit⁻¹ beträgt.

14. Verfahren zur Durchführung einer biochemischen Reaktion mittels eines Moduls nach einem der vorhergehenden Ansprüche, bei welchem während der biochemischen Reaktion die Versorgungsflüssigkeit in der Versorgungskammer (10) nicht mit externem Druck beaufschlagt wird, so daß der molekulare Austausch zwischen der Versorgungskammer (10) und der Systemkammer (12) im wesentlichen auf Diffusion basiert.

15. Verfahren nach Anspruch 14 in Verbindung mit Anspruch 3, bei welchem das produzierende System und/oder die Versorgungsflüssigkeit während der biochemischen Reaktion mittels des Magnetrührelementes (17) gerührt wird.

16. Verfahren nach Anspruch 14, bei welchem der Bioreaktionsmodul während der biochemischen Reaktion geschüttelt wird.

## Claims

1. Disposable bioreaction module for single use for cell-free polypeptide biosynthesis,
having a housing (1) comprising a system chamber (12) and a supply chamber (10), wherein the system chamber (12) contains a producing system during the biochemical reaction and the supply chamber (10) contains a supply liquid during the biochemical reaction and the system chamber (12) and the supply chamber (10) are separated from each other by a semi-permeable membrane (7),
wherein the housing (1) comprises at least two chamber elements (3,5) each having a ring-shaped mounting region (18) and wherein a semi-permeable membrane (7) is sealed and mounted between the ring-shaped mounting regions (18) of the chamber elements (3,5), by sealing means (9) engaging a peripheral marginal section (14) of the membrane (7) in such a manner that the system chamber (12) is defined by a system chamber element (5) and the semi-permeable membrane (7) and the supply chamber is defined by a supply chamber element (3) and the semi-permeable membrane (7).

2. Module according to claim 1 wherein one of the chamber elements (5 or 3 respectively) has a peripheral groove (13) in the mounting region (18) for mounting the semi-permeable membrane (7) and the other chamber element (3 or 5 respectively) has a protruding rib (16) for penetrating into the groove (13) to push the membrane (7) at its peripheral marginal section (14) disposed between the chamber elements (3,5) into the groove.

3. Disposable bioreaction module for single use for cell-free polypeptide biosynthesis,
having a housing (1) comprising a system chamber (12) and a supply chamber (10), wherein the system chamber (12) contains a producing system during the biochemical reaction and the supply chamber (10) contains a supply liquid during the biochemical reaction and the system chamber (12) and the supply chamber (10) are separated from each other by a semi-permeable membrane (7),
wherein
the housing (1) comprises at least two chamber elements (3,5) between which a semi-permeable membrane (7) is sealed and mounted by sealing means (9) engaging a peripheral marginal section (14) of the membrane (7), in such a manner that the system chamber (12) is defined by a system chamber element (5) and the semi-permeable membrane (7) and the supply chamber is defined by a supply chamber element (3) and the semi-permeable membrane (7) and
the at least one supply chamber (10) and/or the system chamber (12) contains a magnetic stirring element (17).

4. Module according to any one of the preceding claims
wherein one of the chamber elements (3,5) has a fill opening (40,41) in a wall (38,39) opposite to the semi-permeable membrane (7) for filling the chamber (10,12) defined by said chamber element (3,5).

5. Module according to claim 4 wherein both the system chamber element (5) and the supply chamber element (3) have a fill opening (40,41) for filling the chamber (10,12) defined by the respective chamber element (3,5), that fill opening being located in a wall (38,39) opposite to the membrane.

6. Module according to claim 5 wherein the walls (38,39) of the system chamber element (5) and of the supply chamber element (3) containing the fill openings (40,41) are each configured as seating surfaces (54,55) for the module (2) and are oriented such that when the module seats on one of these seating surfaces (e.g. 54), the other respective chamber (e.g. 12) can be filled.

7. Module according to any one of claims 4 to 6 wherein the fill opening (40,41) can be closed by a sealing plug (47) which protrudes outwardly from a flap member (49-52) hinged for pivoting on the wall (38,39) opposite to the semi-permeable membrane (7).

8. Module according to any one of claims 4 to 7 wherein the wall (38,39) opposite to the semi-permeable membrane (7) has both a fill opening (40,41) and an additional vent opening (45,46).

9. Module according to any one of the preceding claims
wherein the semi-permeable membrane (7) is a dialysis membrane.

10. Module according to any one of the preceding claims
wherein the molecular cutoff of the membrane (7) is at least 10 kDalton and not more than 20 kDalton.

11. Module according to any one of the preceding claims
wherein the housing comprises a system chamber (12) and two supply chambers (10,11) bordering the system chamber which are separated from the system chamber (12) by a first and a second semi-permeable membrane (7,8), the housing (1) having two supply chamber elements (3,4), wherein the first semi-permeable membrane (7) is mounted between the system chamber element (5) and the first supply chamber element (3) and the second semi-permeable membrane (8) is mounted between the system chamber element (5) and the second supply chamber element (4).

12. Module according to any one of the preceding claims
wherein the ratio of the volume of the system chamber (12) to the total volume of the one or of the plurality of supply chambers (10,11) bordering the system chamber (12) and separated therefrom by means of a semi-permeable membrane, is at least 1:5 and at most 1:20.

13. Module according to any one of the preceding claims
wherein the ratio of the overall exchange surface of the membrane or membranes separating the system chamber from one or more of the supply chambers to the overall volume of the system chamber is at least 0.2 units⁻¹ and at most 4.0 units⁻¹.

14. Method for carrying out a biochemical reaction using a module according to any one of the preceding claims
wherein, during the biochemical reaction, the supply liquid is not subjected to external pressure in the supply chamber (10) so that molecular exchange between the supply chamber (10) and the system chamber (12) is substantially due to diffusion.

15. Method according to claim 14 in combination with claim 3, wherein the producing system and/or the supply liquid is stirred during the biochemical reaction using a magnetic stirring element (17).

16. Method according to claim 14 wherein the bioreaction module is shaken during the biochemical reaction.

## Revendications

1. Module à jeter destiné à des réactions biochimiques et à n'utiliser qu'une seule fois pour la biosynthèse de polypeptides en l'absence de cellules,
comprenant un logement (1) qui renferme une chambre (12) destinée au système et une chambre d'alimentation (10), dans lequel la chambre (12) destinée au système contient, au cours de la réaction biochimique, un système productif, la chambre d'alimentation (10) contient, au cours de la réaction biochimique, un liquide d'alimentation, et la chambre (12) destinée au système et la chambre d'alimentation (10) sont séparées par une membrane semi-perméable (7),
dans lequel, le logement (1) présente au moins deux parties de chambre (3, 5) possédant chacune une zone de fixation annulaire (18) et, entre les zones de fixation annulaires (18) des parties de chambre (3, 5), une membrane semi-perméable (7), qui est fixée de manière étanche à l'aide d'éléments d'étanchéité (9) entourant la membrane (7) dans sa zone marginale périphérique (14), de telle sorte que la chambre (12) destinée au système est délimitée par une partie (5) de la chambre destinée au système et par la membrane semi-perméable (7), et la chambre d'alimentation est délimitée par une partie (3) de la chambre d'alimentation et par la membrane semi-perméable (7).

2. Module selon la revendication 1, dans lequel une des parties de chambre (5, respectivement 3) présente, dans la zone de fixation (18) servant à la fixation de la membrane semi-perméable (7), une rainure périphérique (13) et l'autre partie de chambre (3, respectivement 5) présente une nervure (16) faisant saillie pour pénétrer dans ladite rainure (13), nervure par laquelle la membrane (7) est pressée dans la rainure, dans sa zone marginale périphérique (14) fixée entre les parties de chambre (3, 5).

3. Module à jeter destiné à des réactions biochimiques et à n'utiliser qu'une seule fois pour la biosynthèse de polypeptides en l'absence de cellules,
comprenant un logement (1) qui renferme une chambre (12) destinée au système et une chambre d'alimentation (10), dans lequel la chambre (12) destinée au système contient, au cours de la réaction biochimique, un système productif, la chambre d'alimentation (10) contient, au cours de la réaction biochimique un liquide d'alimentation, et la chambre (12) destinée au système et la chambre d'alimentation (10) sont séparées par une membrane semi-perméable (7),
dans lequel, le logement (1) présente au moins deux parties de chambre (3, 5) entre lesquelles une membrane semi-perméable (7), est fixée de manière étanche à l'aide d'éléments d'étanchéité (9) entourant la membrane (7) dans sa zone marginale périphérique (14), de telle sorte que la chambre (12) destinée au système est délimitée par une partie (5) de la chambre destinée au système et par la membrane semi-perméable (7), et la chambre d'alimentation est délimitée par une partie (3) de la chambre d'alimentation et par la membrane semi-perméable (7) et
ladite au moins une chambre d'alimentation (10) et/ou ladite chambre (12) destinée au système contiennent un élément d'agitation magnétique (17).

4. Module selon l'une quelconque des revendications précédentes, dans lequel au moins une des parties de chambre (3, 5) présente, dans une paroi (38, 39) opposée à la membrane semi-perméable (7), une ouverture de remplissage (40, 41) pour le remplissage des chambres (10, 12) entourées par les parties de chambres (3, 5).

5. Module selon la revendication 4, dans lequel aussi bien la partie (5) de la chambre destinée au système que la partie (3) de la chambre d'alimentation présentent, dans une paroi (38, 39) opposée à la membrane, une ouverture de remplissage (40, 41) pour le remplissage des chambres (10, 12) entourées par les parties de chambres (3, 5).

6. Module selon la revendication 5, dans lequel les parois (38, 39) contenant les ouvertures de remplissage (40, 41), de la partie (5) de la chambre destinée au système et de la partie (3) de la chambre d'alimentation sont réalisées respectivement sous la forme de surfaces de pose (54, 55) pour le module (2) et sont orientées de telle sorte que, lorsque le module est posé sur une surface de pose de ce type (par exemple 54) une chambre en alternance (par exemple 12) peut être remplie.

7. Module selon l'une quelconque des revendications 4 à 6, dans lequel l'ouverture de remplissage (40, 41) peut être fermée à l'aide d'un bouchon de fermeture (47) qui fait saillie par rapport à un élément de basculement (49 - 52) qui est articulé en pivotement à la paroi (38, 39) opposée à la membrane semi-perméable (7).

8. Module selon l'une quelconque des revendications 4 à 7, dans lequel là paroi (38, 39) opposée à la membrane semi-perméable (7) présente, en plus de l'ouverture de remplissage (40, 41), une ouverture d'évacuation supplémentaire (45, 46).

9. Module selon l'une quelconque des revendications précédentes, dans lequel la membrane semi-perméable (7) est une membrane de dialyse.

10. Module selon l'une quelconque des revendications précédentes, dans lequel la limite d'exclusion de la membrane (7) s'élève au minimum à 10 kDalton et au maximum à 20 kDalton.

11. Module selon l'une quelconque des revendications précédentes, dans lequel le logement renferme une chambre (12) destinée au système et deux chambres d'alimentation (10, 11) adjacentes à la chambre destinée au système, qui sont séparées de la chambre (12) destinée au système par une première et par une deuxième membrane semi-perméable (7, 8) et le logement (1) présente deux parties de chambres d'alimentation (3, 4), dans lequel la première membrane semi-perméable (7) est fixée entre la partie (5) de la chambre destinée au système et la partie (3) de la première chambre d'alimentation et la deuxième membrane semi-perméable (8) est fixée entre la partie (5) de la chambre destinée au système et la partie (4) de la deuxième chambre d'alimentation.

12. Module selon l'une quelconque des revendications précédentes, dans lequel la relation entre le volume de la chambre (12) destinée au système et le volume global d'une ou de plusieurs chambres d'alimentation (10, 11) adjacentes à la chambre (12) destinée au système et séparées de cette dernière par une membrane semi-perméable, s'élève au minimum à 1 : 5 et au maximum à 1 : 20.

13. Module selon l'une quelconque des revendications précédentes, dans lequel la relation entre la surface d'échange totale d'une ou de plusieurs membranes délimitant la chambre destinée au système par rapport à une ou plusieurs chambres d'alimentation et le volume total de la chambre destinée au système s'élève au minimum à 0,2 unité⁻¹ et au maximum à 4,0 unités⁻¹.

14. Procédé pour la mise en oeuvre d'une réaction biochimique à l'aide d'un module selon l'une quelconque des revendications précédentes, dans lequel, au cours de la réaction biochimique, le liquide d'alimentation dans la chambre d'alimentation (10) n'est pas soumis à une pression externe, si bien que l'échange moléculaire entre la chambre d'alimentation (10) et la chambre (12) destinée au système est essentiellement basé sur la diffusion.

15. Procédé selon la revendication 14, en liaison avec la revendication 3,
dans lequel le système productif et/ou le liquide d'alimentation sont agités à l'aide de l'élément d'agitation magnétique (17), au cours de la réaction biochimique.

16. Procédé selon la revendication 14, dans lequel le module utilisé pour la - réaction biochimique est secoué au cours de la réaction biochimique.
